(19) Europäisches Patentamt
European Patent Office
Office européen des brevets

(11) Veröffentlichungsnummer: **0 081 206**
**A2**

(12) **EUROPÄISCHE PATENTANMELDUNG**

(21) Anmeldenummer: 82111157.2

(22) Anmeldetag: 02.12.82

(51) Int. Cl.³: C 07 C 125/07
C 07 C 155/08, C 07 C 125/067
C 07 C 103/42, C 07 C 97/10
C 07 C 91/40, A 01 N 47/20
A 01 N 47/30, A 01 N 37/22

(30) Priorität: 09.12.81 DE 3148594

(43) Veröffentlichungstag der Anmeldung: 15.06.83 Patentblatt 83/24

(84) Benannte Vertragsstaaten: AT BE CH DE FR GB IT LI NL

(71) Anmelder: BASF Aktiengesellschaft
Carl-Bosch-Strasse 38
D-6700 Ludwigshafen(DE)

(72) Erfinder: Plath, Peter, Dr.
Bernerweg 24
D-6700 Ludwigshafen(DE)

(72) Erfinder: Schirmer, Ulrich, Dr.
Berghalde 79
D-6900 Heidelberg(DE)

(72) Erfinder: Reissenweber, Gernot, Dr.
Drosselstrasse 15
D-6737 Boehl-Iggelheim(DE)

(72) Erfinder: Wuerzer, Bruno, Dr. Dipl.-Landwirt
Ruedigerstrasse 13
D-6701 Otterstadt(DE)

(72) Erfinder: Retzlaff, Guenter, Dr.
Schillerstrasse 34
D-6725 Roemerberg(DE)

(54) **Anilinderivate, Verfahren zu ihrer Herstellung und ihre Verwendung zur Bekämpfung unerwünschten Pflanzenwuchses.**

(57) Die Erfindung betrifft Anilinderivate der Formel

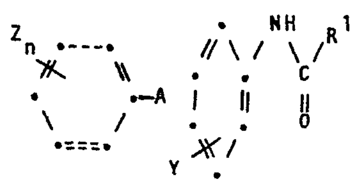

in der
$R^1$, Y, A, Z und n die in der Beschreibung genannten Bedeutungen haben, Verfahren zu ihrer Herstellung und ihre Verwendung zur Bekämpfung unerwünschten Pflanzenwuchses.

EP 0 081 206 A2

# Anilinderivate, Verfahren zu ihrer Herstellung und ihre Verwendung zur Bekämpfung unerwünschten Pflanzenwuchses

Die Erfindung betrifft Anilinderivate, Verfahren zu ihrer Herstellung, Herbizide, die diese Verbindungen als Wirkstoffe enthalten, sowie ein Verfahren zur Bekämpfung unerwünschten Pflanzenwuchses mit diesen Verbindungen.

Es ist bekannt, daß N-Methoxy-N-methyl-N'-phenyl-harnstoffe herbizid wirksam sind (DE-OS 28 28 417).

Es wurde gefunden, daß Anilinderivate der Formel

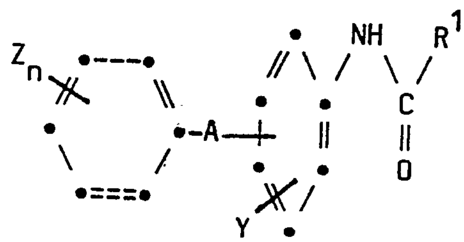

(I),

in der

$R^1$ Alkoxy, Alkylthio oder gegebenenfalls durch Halogen oder Alkoxy mit 1 bis 4 Kohlenstoffatomen substituiertes Alkyl mit jeweils bis zu 4 Kohlenstoffatomen oder Cycloalkyl mit 3 bis 6 Kohlenstoffatomen oder den Rest 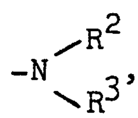, wobei $R^2$ und $R^3$ unabhängig voneinander Wasserstoff, Alkyl oder Alkoxy mit jeweils bis zu 4 Kohlenstoffatomen oder Cycloalkyl mit 3 bis 6 Kohlenstoffatomen bedeuten oder $R^2$ und $R^3$ zusammen eine gegebenenfalls durch Methyl substituierte und gegebenenfalls Sauerstoff als Kettenglied enthaltende Alkylenkette mit bis zu 6 Kohlenstoffatomen bedeuten,

H/HB

Y Wasserstoff, Halogen, Methyl, Methoxy oder Trifluormethyl,

A eine die Carbonyl- oder die Carbinolgruppe der Formel >CH-OH enthaltende Alkylenkette mit 2 bis 9 Kohlenstoffatomen, die durch Methyl substituiert sein kann,

Z Wasserstoff, Halogen, Alkyl, Alkoxy, Halogenalkyl oder Halogenalkoxy mit jeweils bis zu 6 Kohlenstoffatomen, Phenyl oder Phenoxy und

n die Zahlen 1, 2 oder 3 bedeuten und in der anstelle des Restes 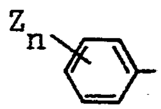 ein gegebenenfalls durch Halogen, Alkyl oder Alkoxy mit jeweils bis zu 4 Kohlenstoffatomen substituierter Naphthylrest stehen kann.

eine herbizide Wirkung haben und für eine Reihe von Kulturpflanzen ein hohes Maß an Verträglichkeit besitzen.

Die Substituenten in Formel I können die folgenden Bedeutungen haben:

$R^1$ bedeutet Alkoxy, Alkylthio oder gegebenenfalls durch Halogen oder Alkoxy mit 1 bis 4 Kohlenstoffatomen substituiertes Alkyl mit jeweils bis zu 4 Kohlenstoffatomen, beispielsweise Methoxy, Ethoxy, n-Propoxy, Isopropoxy, sec-Butoxy, tert.-Butoxy, Methylthio, Ethylthio, n-Propylthio, sec.-Butylthio, tert.-Butylthio, Methyl, Ethyl, n--Propyl, n-Butyl, sec.-Butyl, tert.-Butyl, Isobutyl, Chlormethyl, 1,1-Dichlorethyl, Dichlormethyl, Methoxymethyl, 1-Methoxyethyl, 2-Methoxyethyl, Cycloalkyl mit 3 bis 6 Kohlenstoffatomen, beispielsweise Cyclopropyl,

Cyclopentyl, Cyclohexyl oder den Rest $-N\begin{smallmatrix}R^2\\R^3\end{smallmatrix}$, wobei $R^2$ und $R^3$ unabhängig voneinander Wasserstoff, Alkyl oder Alkoxy mit jeweils bis zu 4 Kohlenstoffatomen, beispielsweise Methyl, Ethyl, Isopropyl, n-Butyl, sec-Butyl, Isobutyl, tert.-Butyl, Methoxy, Ethoxy oder Cycloalkyl mit 3 bis 6 Kohlenstoffatomen, beispielsweise Cyclopropyl, Cyclopentyl, Cyclohexyl oder $R^2$ und $R^3$ zusammen eine gegebenenfalls durch Methyl substituierte und gegebenenfalls Sauerstoff als Kettenglied enthaltende Alkylenkette mit bis zu 6 Kohlenstoffatomen, beispielsweise Trimethylen, Tetramethylen, 1,4-Dimethyltetramethylen, Pentamethylen, Hexamethylen, 3-Oxapentamethylen, 1-Oxa-pentamethylen, 1-Oxa-tetramethylen, bedeuten.

Y bedeutet Wasserstoff, Halogen, beispielsweise Fluor, Chlor, Brom, sowie Methyl, Methoxy oder Trifluormethyl.

A steht in meta- oder para-Stellung zu der Gruppe $-NH-CO-R^1$ und bedeutet eine die Carbonyl- oder die Carbinolgruppe der Formel $>CH-OH$ enthaltende Alkylenkette mit 2 bis 9 Kohlenstoffatomen, die durch Methyl substituiert sein kann, beispielsweise eine $C_2$-Kette, wie $-CO-CH_2-$ oder $-CH_2-CO$, sowie die daraus durch Reduktion erhältlichen Carbinole $-CH(OH)-CH_2-$ bzw. $-CH_2-CH(OH)-$, eine $C_3$-Kette, wie $-CO-(CH_2)_2-$, $-(CH_2)_2-CO-$, $-CO-CH(CH_3)-CH_2-$, $-CH_2-CH(CH_3)-CO-$ sowie die daraus erhältlichen Carbinole, eine $C_4$-Kette wie $-CO-(CH_2)_3-$, $-(CH_2)_3-CO-$, $-CH_2-CO-(CH_2)_2-$, $-(CH_2)_2-CO-(CH_2)-$ sowie die daraus erhältlichen Carbinole, eine $C_5$-Kette, wie $-CO-(CH_2)_4-$, $-(CH_2)_4-CO-$, $-(CH_2)_2-CO-(CH_2)_2-$, $-CO-(CH_2)_2-CH(CH_3)-CH_2-$, $-CH_2-CH(CH_3)-(CH_2)_2-CO-$, sowie die daraus erhältlichen Carbinole, eine $C_6$-Kette, wie $-CO-(CH_2)_5-$, $-(CH_2)_5-CO-$, $-CH_2-CO-(CH_2)_4-$, $-(CH_2)_4-CO-CH_2-$,

$-CH_2-CO-(CH_2)_2-CH(CH_3)-CH_2-$, $-CH_2-CH(CH_3)-(CH_2)_2-CO-CH_2-$, sowie die daraus erhältlichen Carbinole, eine $C_7$-Kette, wie $-CO-(CH_2)_6-$, $-(CH_2)_6-CO-$, $-(CH_2)_3-CO-(CH_2)_3-$, $-(CH_2)_2-CO-(CH_2)_4-$, $-(CH_2)_4-CO-(CH_2)_2-$, $CH_2-CH(CH_3)-(CH_2)_2-CO-(CH_2)_2-$, $-(CH_2)_2-CO-(CH_2)_2-CH(CH_3)-CH_2-$, sowie die daraus erhältlichen Carbinole, eine $C_8$-Kette,wie $-CO-(CH_2)_7-$, $(CH_2)_7-CO-$ sowie die daraus erhältlichen Carbinole oder eine $C_9$-Kette, wie $-CO(CH_2)_8-$, $-(CH_2)_8-CO-$, $-(CH_2)_4-CO-(CH_2)_4-$, $-CH_2-CH(CH_3)-(CH_2)_2-CO-(CH_2)_4-$, $-(CH_2)_4-CO-(CH_2)_2-CH(CH_3)-CH_2-$ sowie die daraus erhältlichen Carbinole.

Z bedeutet Wasserstoff, Halogen, beispielsweise Fluor, Chlor, Brom, Alkyl, Alkoxy, Halogenalkyl oder Halogenalkoxy mit jeweils bis zu 6 Kohlenstoffatomen, beispielsweise Methyl, Ethyl, Isopropyl, tert.-Butyl, n-Hexyl, Methoxy, Ethoxy, Isopropoxy, tert.-Butoxy, n-Hexoxy, Trifluormethyl, 1,1,2-Trifluor-2-chlorethoxy, Difluormethoxy, Phenyl oder Phenoxy. Z steht in ortho-, meta- oder para-Stellung zu A. n ist vorzugsweise 1, kann jedoch auch 2 oder 3 bedeuten, insbesondere, wenn Z für Fluor, Chlor, Brom oder Methyl steht.

In Formel I kann der Rest 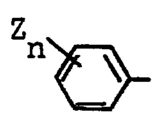 auch durch einen α- oder ß-Naphthylrest, der durch Halogen, wie Chlor oder Brom, oder durch Alkyl oder Alkoxy mit jeweils bis zu 4 Kohlenstoffatomen, wie Methyl, Ethyl, Methoxy, Ethoxy, Isopropyl, tert.-Butoxy, substituiert sein kann, ersetzt sein.

Bevorzugte Verbindungen der Formel I sind solche, bei denen $R^1$ den Rest 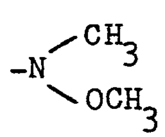, Y Wasserstoff oder Halogen, insbesondere Chlor, A den Rest $-CO-(CH_2)_2-$ in para-Stellung zu der Gruppe $-NH-CO-R^1$, Z Halogen, insbesondere Chlor, oder Alkyl mit bis zu 4 Kohlenstoffatomen, insbesondere Methyl, und n 1 bedeuten.

Man erhält die Anilinderivate der Formel I durch Umsetzung von Aminen der Formel

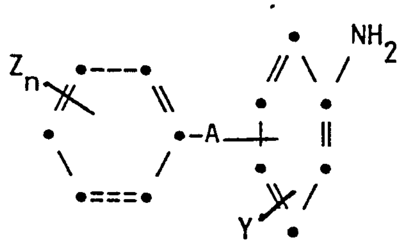
(II),

in der
A, Y, Z und n die obengenannten Bedeutungen haben, mit Verbindungen der Formel

$$R^1 - CO - X \qquad (III),$$

in der $R^1$ die obengenannten Bedeutungen hat und X eine Abgangsgruppe, vorzugsweise Halogen, wie Chlor oder Brom, oder $R^1$-CO-O- bedeutet.

Die Umsetzung wird in einem inerten organischen Lösungsmittel durchgeführt. Geeignet sind Ether, wie Tetrahydrofuran, Dimethoxyethan, Diethylether, Methyl-tert.-butyl-ether, Ester wie Essigsäureethylester, gegebenenfalls chlorierte aliphatische oder aromatische Kohlenwasserstoffe, wie Toluol, Dichlormethan, Pyridin. Auch Gemische dieser Lösungsmittel können verwendet werden. Die Menge an Lösungsmittel, bezogen auf Amin der Formel II beträgt 100 bis 5000 Gew.%.

Zweckmäßigerweise wird die Umsetzung in Gegenwart eines Säureakzeptors durchgeführt. In Betracht kommen Alkalihydroxide, Alkalicarbonate, Alkalihydrogencarbonate, Erdalkalihydroxide, Erdalkalicarbonate, Erdalkalihydrogencarbonate, Erdalkalioxide oder Amine, beispielsweise Natriumhydrogencarbonat, Kaliumcarbonat, Triethylamin, Pyridin, N,N-Dimethylanilin, N,N-Dimethyl-N-cyclohexylamin, Chinolin. Die Menge an Säureakzeptor beträgt 1 bis 4 Mol, bezogen auf 1 Mol Verbindung der Formel III.

Die Ausgangsstoffe der Formeln II und III werden bevorzugt in äquimolarem Verhältnis miteinander umgesetzt. Die Reaktionstemperatur liegt zwischen 20 und 80°C, vorzugsweise zwischen 20 und 30°C.

Die Amine der Formel II sind neu. Sie werden nach bekannten Verfahren durch Aldolkondensation und anschließende Hydrierung erhalten. Man setzt beispielsweise gegebenenfalls substituierte Acetophenone mit Nitrobenzaldehyden zu Nitrochalkone um

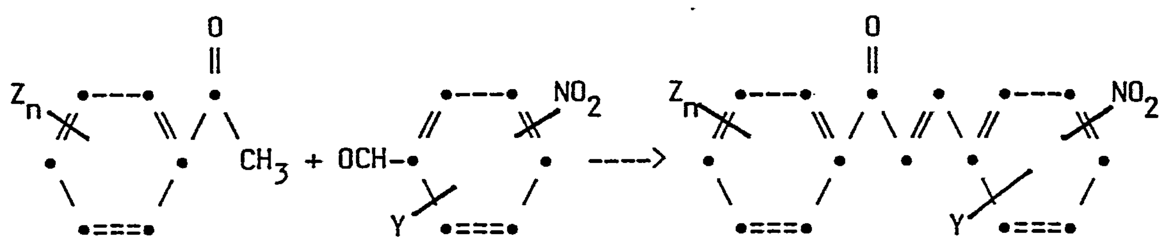

und hydriert die so erhaltenen Nitrochalkone in an sich bekannter Weise in einem organischen Lösungsmittel, wie Methanol, Tetrahydrofuran, Essigsäure oder Essigsäuremethylester, in Gegenwart von Palladium auf Kohle oder Raney-Nickel bei Temperaturen zwischen 20 und 150°C, vorzugsweise zwischen 20 und 80°C, gegebenenfalls unter Druck. Y, Z und n haben dabei die obengenannten Bedeutungen.

Amine der Formel II, bei denen A $-CO-(CH_2)_4-$ oder $-CO-(CH_2)_2-CH(CH_3)-CH_2-$ bedeutet, werden dadurch erhalten, daß man gegebenenfalls substituierte Acetophenone mit Nitrozimtaldehyden kondensiert, beispielsweise nach folgendem Schema:

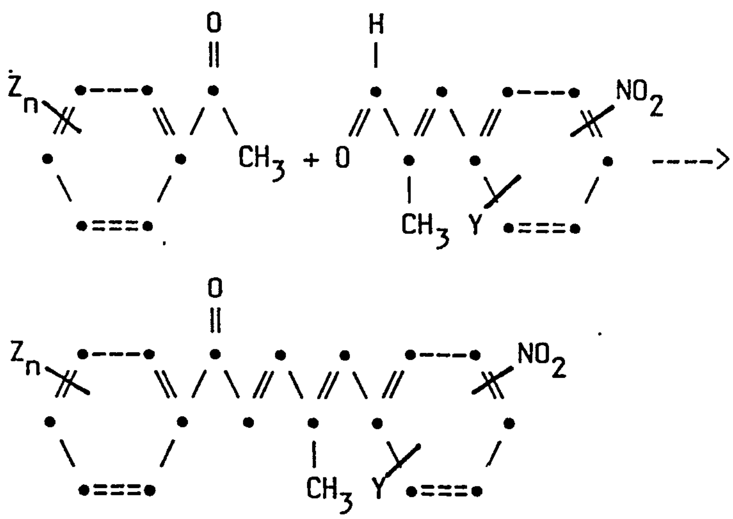

Eine weitere Synthesemöglichkeit für Amine der Formel II besteht in der Kondensation von gegebenenfalls substituierten Benzaldehyden oder Zimtaldehyden mit Nitroacetophenonen nach folgendem Reaktionsschema:

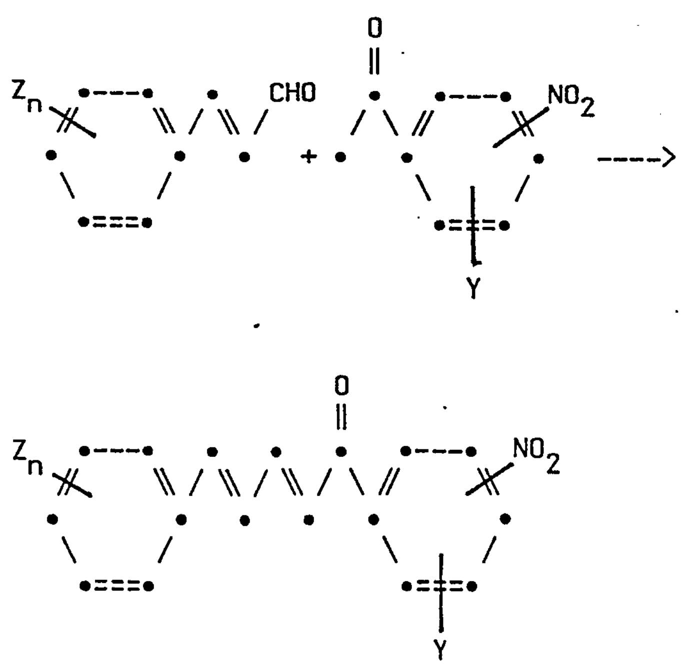

Es kann auch vorteilhaft sein, von gegebenenfalls substituiertem Benzalaceton auszugehen und dieses mit Nitrobenzaldehyden oder Nitrozimtaldehyden zu kondensieren:

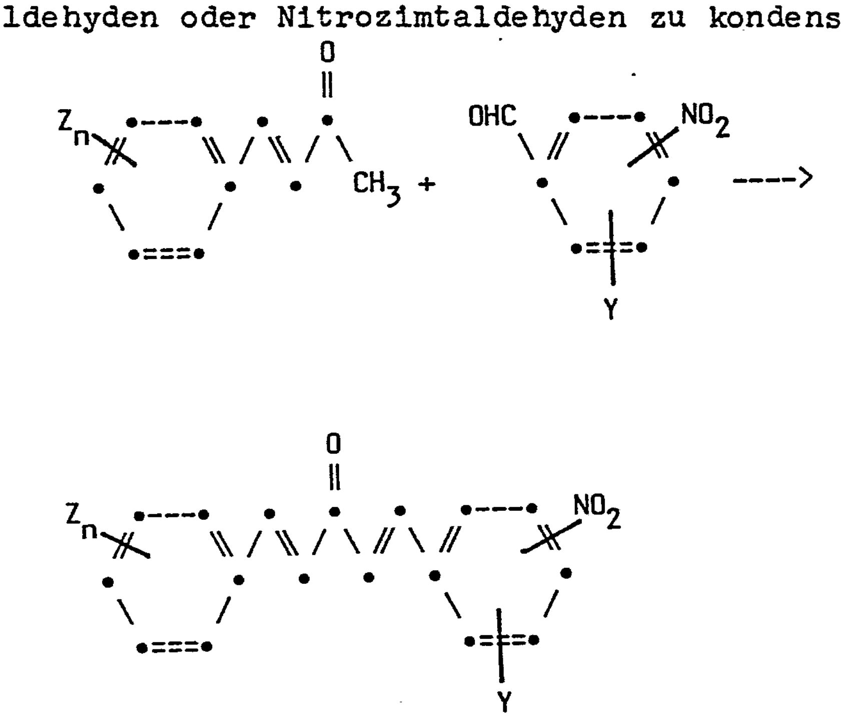

Bei allen drei Verfahrensvarianten wird anschließend katalytisch hydriert.

Amine der Formel II, bei denen A für den Rest $-(CH_2)_p-CO-$ steht, wobei p eine ganze Zahl von 1 bis 8 bedeutet, können durch Acylierung von Carbonsäurechloriden der Formel

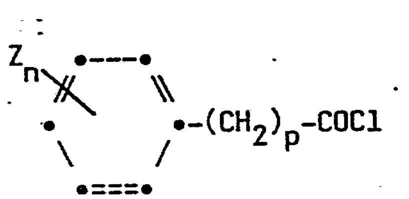

(V),

in der Z, n und p die obengenannten Bedeutungen haben, nach Friedel-Crafts nach folgendem Schema

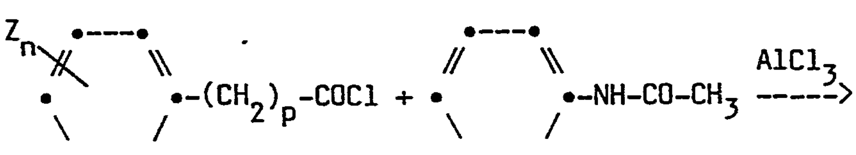

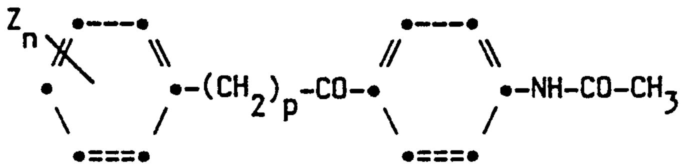

und anschließende Verseifung erhalten werden.

Durch Umsetzung von m- oder p-Nitrophenyl-alkansäurechloriden mit Aromaten der Formel 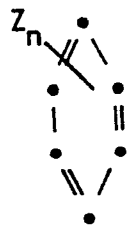, wobei Z und n die obengenannten Bedeutungen haben, erhält man folgende Nitrophenyl-alkylarylketone:

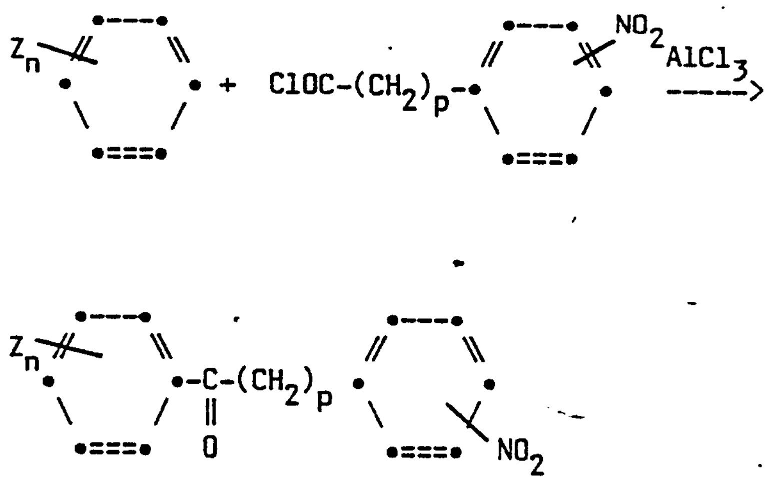


Man kann andererseits auch ω-Phenylalkansäurechloride nach Friedel-Crafts mit 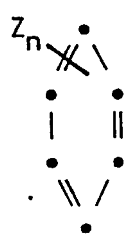 umsetzen und die so erhaltenen ω,α-Diaryl-alkyl-ketone nachträglich in para-Stellung zu $-(CH_2)_p-$ nitrieren:

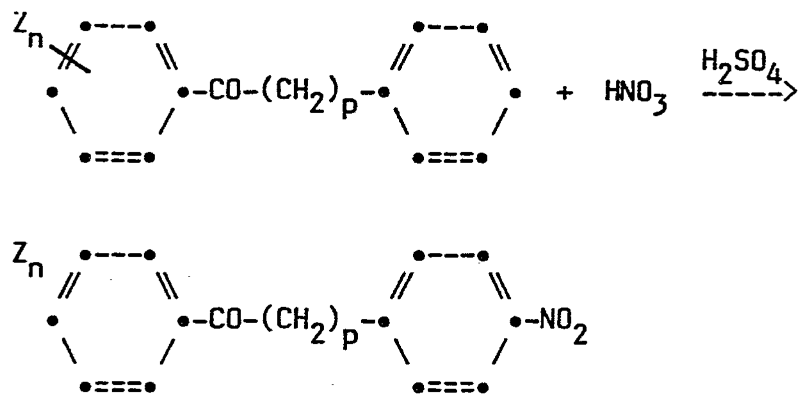


Die so erhaltenen Nitrophenyl-ketone lassen sich katalytisch zu Anilinen der Formel VI hydrieren:

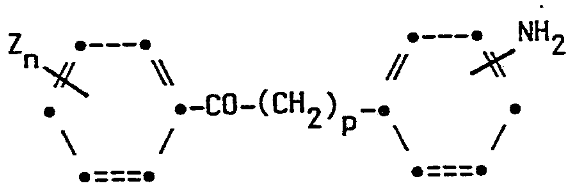
(VI).

In diesen Formeln bedeutet p eine ganze Zahl von 1 bis 8 (G. Olah, Friedel-Crafts and Related Reactions, Vol. III, Part I, S. 23, 24, 1393ff, Interscience Publishers, New York, 1964).

Die Wasserstoffaufnahme bei der Hydrierung der vorgenannten gesättigten oder ungesättigten Nitrophenylketone erfolgt stufenweise und kann entweder nach Erreichen der gesättigten Anilinoketon-Stufe abgebrochen werden oder bis zur Anilinocarbinolstufe weitergeführt werden.

Die Hydrierung erfolgt in an sich bekannter Weise in Gegenwart von Raney-Nickel oder vorzugsweise von Palladium auf Aktivkohle. In diesem Fall gelingt die Hydrierung zum gesättigten Anilinoketon bereits bei Temperaturen von 0 bis 30°C, vorzugsweise bei 25°C und bei einem Druck von 1,01 bis 20 bar, bevorzugt bei 1,1 bar. Verlängerung der Reaktionsdauer, Erhöhung der Hydriertemperatur und/oder Druckerhöhung bis zu 50 bar führt zur Reduktion der Ke-

togruppe zur Carbinolgruppe. Die Hydrierung der Ketogruppe erfolgt umso leichter, je näher die Ketogruppe in Nachbarschaft zu einem der beiden Benzolringe steht (Houben-Weyl, Methoden der Org. Chemie, Bd. 4/1c, S. 13ff, Georg-Thieme-Verlag, Stuttgart, 1980).

Die folgenden Beispiele veranschaulichen die Synthese der Amine der Formel II:

Beispiel A

1-(4-Methyl-phenyl)-3-(4-amino-phenyl)-n-propan-1-on

In einer 2l-Rührapparatur mit aufgesetztem Wasserabscheider wird eine Mischung aus 272 g 4-Nitrobenzaldehyd, 255 g 4-Methylacetophenon und 40 g Bortrioxid mit 400 ml Xylol zum Sieden erhitzt. Nach 8 Stunden haben sich 32 ml Wasser abgeschieden. Man läßt abkühlen und gießt die Reaktionslösung in 1 l Toluol. Der ausgefallene Feststoff wird abgesaugt und unter vermindertem Druck getrocknet. Man erhält 391 g (Ausbeute: 81 % d.Th.) eines gelben Feststoffs vom Fp. 163-165°C. 189 g der so erhaltenen Nitroverbindung werden in 2 l Tetrahydrofuran gelöst und mit 5 g 10 proz. Pd auf Tierkohle (Degussa Typ E 10 N) versetzt. Anschliessend wird (nach Spülen mit Stickstoff) bei 20°C und geringem Überdruck (1,1 bar) hydriert, bis 64,5 l Wasserstoff aufgenommen wurden. Danach wird die Wasserstoffzufuhr unterbrochen, der Katalysator durch Filtration abgetrennt und das Lösungsmittel unter vermindertem Druck abgezogen. Der Rückstand wird mit Diisopropylether verrührt und abgesaugt. Man erhält 159 g (Ausbeute: 94 % d.Th.) eines gelblichen kristallinen Feststoffs vom Fp. 59-61°C.

Beispiel B

1-(4-Methyl-phenyl)-5-(4-amino-phenyl)-n-pentan-3-on

Zu einer Mischung aus 80 g 1-(4-Methylphenyl)-2-buten-3--on, 83 g 4-Nitrobenzaldehyd und 600 ml Ethanol tropft man bei 25°C 15 g einer 10 %igen NaOH-Lösung unter Eiskühlung. Nach Beendigung der Zutropfens wird noch 1 Std. lang bei 25°C gerührt. Danach saugt man das Reaktionsprodukt auf einer Nutsche ab und kristallisiert den erhaltenen Feststoff aus Essigsäureethylester um. Man erhält 135 g 1-(4-Methylphenyl)-5-(4'-nitrophenyl)-1,4--pentadien-3-on vom Fp. 182-184°C (Ausbeute: 92 % d.Th.).

60 g des so erhaltenen Pentadienons werden in 2 l Tetrahydrofuran gelöst und mit 6 g 10 % Pd auf Tierkohle (Degussa Typ E 10 N) versetzt. Dann wird die Hydrierapparatur mit Stickstoff gespült. Anschließend wird bei 22 bis 26°C bei 1,1 bar innerhalb 2 1/2 Stunden hydriert, wobei 24 l Wasserstoff aufgenommen werden. Nach Abtrennung des Katalysators durch Filtration wird das Filtrat über $MgSO_4$ getrocknet und anschließend eingedampft. Man erhält 49 g (Ausbeute: 89,5 % d.Th.) eines Öls, das im IR-Spektrum die $-NH_2$- und die CO-Gruppe zeigt und folgende Struktur hat:

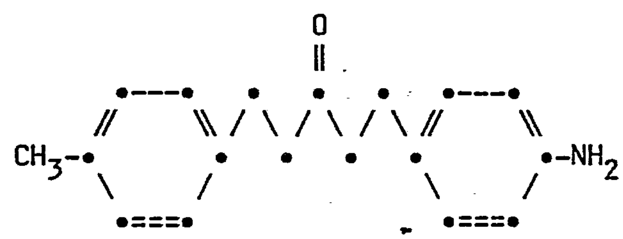

Beispiel C

1-(4-Amino-phenyl)-7-phenyl-n-heptan-3-on

Zu einer Mischung aus 46,5 g Cinamalaceton und 45,4 g p--Nitrobenzaldehyd in 160 ml Ethanol tropft man unter leichter Kühlung bei 25°C 16,2 g 10 proz. NaOH-Lösung zu. Die

Reaktionsmischung wird nach kurzer Zeit klar und scheidet später einen gelben Feststoff ab. Nach einstündigem Rühren wird der Feststoff durch Absaugen isoliert und mit Diethylether nachgewaschen. Nach dem Trocknen unter vermindertem Druck erhält man 76 g (Ausbeute: 91 % d.Th.) 1-(4-Nitrophenyl)-7-phenyl-1,4,6-heptatrien-3-on vom Fp. 168-170°C.

35 g dieses Produkts werden in Gegenwart von 3,5 g 10 % Pd auf Tierkohle in 350 ml Tetrahydrofuran bei 1,1 bar hydriert, bis 15 l Wasserstoff aufgenommen sind. Nach Abtrennung des Katalysators durch Filtration und Trocknen des Filtrats über $MgSO_4$ verbleiben nach Abziehen des Lösungsmittels 26 g (Ausbeute: 80,5 % d.Th.) eines Öls, das im IR-Spektrum die $-NH_2$-Gruppe und die CO-Gruppe zeigt und folgende Struktur hat:

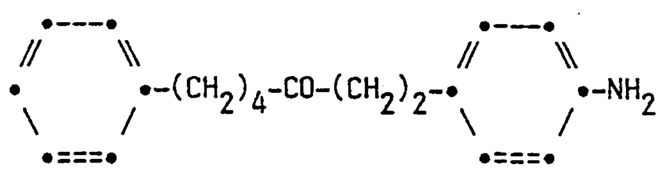

<u>Beispiel D</u>

1-(4-Amino-phenyl)-3-methyl-5-(4-methylphenyl)-n-pentan-5-on

In eine Mischung aus 95,5 g 4-Nitro-α-methyl-zimtaldehyd und 74 g 4-Methylacetophenon in 500 ml Ethanol tropft man bei 25 bis 30°C 25 ml einer 5 %igen NaOH-Lösung. Man beobachtet Selbsterwärmung bis 35°C. Nach 2 Stunden Rühren bei 35 bis 40°C läßt man abkühlen, saugt den ausgefallenen Feststoff ab und wäscht mit kaltem Methanol nach. Nach dem Trocknen unter vermindertem Druck erhält man 124 g (Ausbeute: 83 % d.Th.) 1-(4-Nitrophenyl)-3-methyl-5-(4'-Methylphenyl)-2,4-pentadien-5-on vom Fp. 149-151°C.

Die katalytische Hydrierung mit einem 10 proz. Pd-Katalysator auf Tierkohle erfolgt in Tetrahydrofuran analog Beispiel C. Das Produkt enthält nach der Hydrierung zwei Stoffe, die durch Chromatographie an Kieselgel (Eluens: Essigsäureethylester/Cyclohexan = 1:1) getrennt werden. Aus 20 g des Hydrierproduktes werden 7 g 1-(4-Amino-phenyl)-3-methyl-5-(4-methylphenyl)-n-pentan-5-on erhalten. Die zweite Substanz ist das entsprechende Carbinol der Summenformel $C_{19}H_{25}NO$.

Beispiel E

1-(4-Methyl-phenyl)-3-(4-amino-phenyl)-n-propan-1-ol

60 g des nach Beispiel A hergestellten 1-(4-Methylphenyl)-3-(4-amino-phenyl)-n-propan-1-on werden in 1 l Tetrahydrofuran in Gegenwart von 5 g 10 % Pd auf Kohle (Wasserstoffaufnahme: 5,5 l) bei 1,1 bar hydriert. Nach Abtrennung des Katalysators und Eindampfen der Reaktionslösung erhält man ein Öl, das nach Anreiben mit Diisopropylether kristallisiert. Nach dem Trocknen der weißen Kristalle erhält man 53 g (Ausbeute: 88 % d.Th.) Carbinol vom Fp. 63-65°C.

Beispiel F

1-(3-Amino-phenyl)-5-phenyl-n-pentan-1-ol

Analog Beispiel D werden 33 g 3-Nitroacetophenon und 26,2 g Zimtaldehyd in 200 ml Ethanol in Gegenwart von 10 ml 5 %iger NaOH-Lösung zu 1-(3-Nitrophenyl)-5-phenyl-2,4-pentadien-1-on kondensiert. Ausbeute: 38 g (71 % d. Th.); Fp. 127-129°C.

Die Hydrierung dieses Ketons bis zur Beendigung der $H_2$-Aufnahme analog Beispiel E liefert nach der Aufarbeitung

28,5 g (Ausbeute: 79 % d.Th.) eines weißen kristallinen Feststoffs vom Fp. 58-60°C.

In analoger Weise wurden folgende Amine der Formel II synthetisiert:

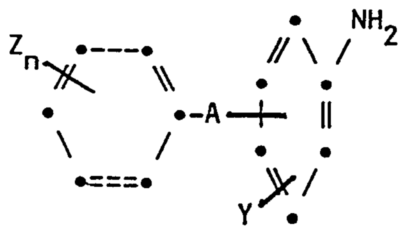

| Stellung von A | A | Y | $Z_n$ | Fp [°C] |
|---|---|---|---|---|
| 3 | $-(CH_2)_2-CO-$ | H | H | 85- 87 |
| 3 | $-CO-(CH_2)_2-$ | H | 4-Cl | 66- 69 |
| 3 | $-(CH_2)_2-CO-$ | H | $4-CH_3$ | |
| 3 | $-(CH_2)_2-CO-$ | H | 4-Cl | |
| 3 | $-(CH_2)_2-CO-$ | H | $3-CF_3$ | 134-136 |
| 3 | $-(CH_2)_2-CO-$ | H | $3,4-Cl_2$ | 97-100 |
| 3 | $-CO-(CH_2)_2-$ | 4-Br | H | |
| 3 | $-CO-(CH_2)_2-$ | H | $2-CH_3$ | |
| 3 | $-CO-(CH_2)_2-$ | H | $3-CH_3$ | |
| 4 | $-CO-(CH_2)_2-$ | H | H | |
| 4 | $-CO-(CH_2)_2-$ | H | 4-Cl | |
| 4 | $-CO-(CH_2)_2-$ | H | $2,4,6-(CH_3)_3$ | Öl; $Kp_{0,3}$ = 187°C |
| 4 | $-CO-(CH_2)_2-$ | 3-Cl | $4-CH_3$ | |
| 4 | $-CO-(CH_2)_2-$ | $3-CH_3$ | $4-CH_3$ | |
| 4 | $-CO-(CH_2)_2-$ | $3-OCH_3$ | $4-CH_3$ | |
| 4 | $-CO-(CH_2)_2-$ | $3-CF_3$ | $4-CH_3$ | |
| 4 | $-CO-(CH_2)_2-$ | H | 3-Cl | |
| 4 | $-CO-(CH_2)_2-$ | H | $4-CF_3$ | |
| 4 | $-CO-(CH_2)_2-$ | H | $4-C_6H_5$ | |
| 4 | $-(CH_2)_2-CO-$ | H | H | |
| 4 | $-(CH_2)_2-CO-$ | H | $4-CH_3$ | 113-115 |
| 4 | $-(CH_2)_2-CO-$ | H | 4-Cl | 103-106 |

| Stellung von A | A | Y | $Z_n$ | Fp [°C] |
|---|---|---|---|---|
| 4 | $-(CH_2)_2-CO-$ | H | $3-OC_6H_5$ | |
| 4 | $-(CH_2)_2-CO-$ | H | $4-O-n-C_6H_{13}$ | |
| 4 | $-CO-CH(CH_3)-CH_2-$ | H | $4-CH_3$ | Öl, $Kp_{0,3}$ = 180-187°C |
| 4 | $-CO-CH(CH_3)-CH_2-$ | H | 4-Cl | Öl |
| 4 | $-CO-(CH_2)_2-CH(CH_3)-CH_2-$ | H | H | Öl |
| 3 | $-CO-(CH_2)_2-CH(CH_3)-CH_2-$ | H | $4-CH_3$ | Öl |
| 3 | $-(CH_2)_2-CO-(CH_2)_2-$ | H | H | Öl |
| 3 | $-(CH_2)_2-CO-(CH_2)_2-$ | H | $4-CH_3$ | Öl |
| 4 | $-(CH_2)_4-CO-(CH_2)_2-$ | H | $4-CH_3$ | Öl |
| 3 | $-CH_2-CO-$ | H | $4-CH_3$ | |
| 3 | $-CO-CH_2-$ | H | $4-CH_3$ | |
| 4 | $-CH_2-CO-$ | H | " | |
| 4 | $-CO-CH_2-$ | H | " | |
| 4 | $-CO-(CH_2)_3-$ | H | " | |
| 4 | $-CO-CH_2-CH(CH_3)-$ | H | " | |
| 3 | $-CH(CH_3)-CH_2-CO-$ | H | " | |
| 4 | $-C(CH_3)_2-CH_2-CO-$ | H | " | |
| 3 | $-C(CH_3)_2-CH_2-CO-$ | H | " | |
| 4 | $-CO-(CH_2)_4-$ | H | " | |
| 4 | $-CO-(CH_2)_5-$ | H | " | |
| 4 | $-CO-(CH_2)_6-$ | H | " | |
| 4 | $-CO-(CH_2)_7-$ | H | " | |

| Stellung von A | A | Y | $Z_n$ | Fp [°C] |
|---|---|---|---|---|
| 4 | $-CO-(CH_2)_8-$ | H | $4-CH_3$ | |
| 3 | $-CH(OH)-(CH_2)_2-$ | H | H | Öl |
| 3 | $-CH(OH)-(CH_2)_2-$ | H | 4-F | Öl |
| 3 | $-CH(OH)-(CH_2)_2-$ | H | $4-CH_3$ | |
| 4 | $-CH(OH)-(CH_2)_2-$ | H | $4-CH_3$ | 63- 65 |
| 4 | $-CH(OH)-(CH_2)_2-$ | H | 4-Cl | |
| 4 | $-CH(OH)-(CH_2)_2-$ | H | $4-CF_3$ | |
| 4 | $-CH(OH)-(CH_2)_3-$ | H | $4-CH_3$ | |
| 4 | $-CH(OH)-CH(CH_3)-CH_2-$ | H | 4-Cl | Öl, $Kp_{0,3}$ = 210-220°C |
| 4 | $-(CH_2)_2-CH(OH)-(CH_2)_2-$ | H | H | Öl |
| 3 | $-(CH_2)_2-CH(OH)-(CH_2)_2-$ | H | $4-CH_3$ | Öl |
| 4 | $-CH(OH)-CH_2-CH(CH_3)-$ | H | " | |
| 3 | $-CH(CH_3)-CH_2-CH(OH)-$ | H | " | |
| 4 | $-C(CH_3)_2-CH_2-CH(OH)-$ | H | " | |
| 3 | $-C(CH_3)_2-CH_2-CH(OH)-$ | H | " | |
| 4 | $-CH(OH)-(CH_2)_4-$ | H | " | |
| 4 | $-CH(OH)-(CH_2)_5-$ | H | " | |
| 4 | $-CH(OH)-(CH_2)_6-$ | H | " | |
| 4 | $-CH(OH)-(CH_2)_7-$ | H | " | |
| 4 | $-CH(OH)-(CH_2)_8-$ | H | " | |
| 3 | $-(CH_2)_2-CH(OH)-$ | H | $3-CF_3$ | Öl, $Kp_{0,3}$ = 240°C |
| 3 | $-(CH_2)_2-CO-$ | H | 3-Cl | Öl |

0081206

| Stellung von A | A | Y | $Z_n$ | Fp [°C] |
|---|---|---|---|---|
| 4 | $-CO-(CH_2)_2-$ | H | 4-F | Öl |
| 3 | $-CO-(CH_2)_2-$ | H | $4-OCH_3$ | 98-100 |
| 3 | $-CO-(CH_2)_2-$ | H | 4-F | 64- 66 |

| Stellung von A | A | Y | $Z_n$ 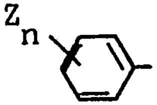 | Fp [°C] |
|---|---|---|---|---|
| 3 | $-CO-(CH_2)_2-$ | H | α-Naphthyl | |
| 3 | $-CO-(CH_2)_2-$ | H | β-Naphthyl | |
| 4 | $-CO-(CH_2)_2-$ | H | α-Naphthyl | |
| 4 | $-CO-(CH_2)_2-$ | H | β-Naphthyl | |

Anilinderivate der Formel I, bei denen $R^1$ Alkoxy oder Alkylthio oder den Rest 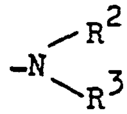 und A eine Carbonyl enthaltende Alkylenkette mit 2 bis 9 Kohlenstoffatomen, die durch Methyl substituiert sein kann, bedeuten und Y, Z und n die obengenannten Bedeutungen haben, erhält man ebenfalls durch Umsetzung von Isocyanaten der Formel

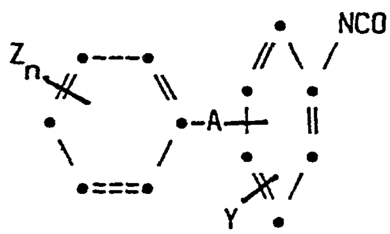

(IV),

in der Y, A, Z und n die vorstehend genannten Bedeutungen haben, mit einer Verbindung der Formel $R^1H$, in der $R^1$ die vorstehend genannten Bedeutungen hat, bei einer Temperatur zwischen 0 und 150, vorzugsweise zwischen 10 und 50°C.

Die Umsetzung wird in einem unter den Reaktionsbedingungen inerten Lösungsmittel durchgeführt. Geeignet sind Ether, wie Diethylether, Methyl-tert.-butyl-ether, Tetrahydrofuran, Ester, wie Essigsäureethylester, gegebenenfalls chlorierte aliphatische oder aromatische Kohlenwasserstoffe, wie Ligroin, Toluol, Cyclohexan, Benzin, Dichlormethan, Chloroform, Chlorbenzol, o-, m-, p-Dichlorbenzol, Nitrobenzol, Ketone, wie Aceton, Nitrile, wie Acetonitril oder Dimethylformamid. Auch Gemische dieser Lösungsmittel können verwendet werden.

Die Umsetzung der Isocyanate der Formel IV mit Alkoholen, Mercaptanen oder Aminen der Formel $R^1H$ kann gegebenenfalls durch Zugabe eines für Isocyanatreaktionen gebräuch-

lichen Katalysators beschleunigt werden, z.B. durch tert.-Amine, wie Triethylamin, 1,4-Diaza-bicyclo[2,2,2]octan, Stickstoffheterocyclen, wie Pyridin oder 1,2-Dimethylimidazol oder organische Zinnverbindungen, wie Dibutylzinndiacetat, Dimethylzinndichlorid.

Zur Herstellung der Isocyanate gemäß Formel IV setzt man die Amine der Formel III mit Phosgen um (Liebigs Ann.Chem. 562, 75ff, (1949)).

Die folgenden Beispiele erläutern die Herstellung der Anilinderivate der Formel I:

Beispiel 1

N-[4-(3-(4-methyl-phenyl)-n-propan-3-on-yl)-phenyl]-N'-methyl-N'-methoxy-harnstoff

Zu einer Lösung von 36 g des nach Beispiel A hergestellten 1-(4-Methyl-phenyl)-3-(4-amino-phenyl)-n-propan-1-on in 250 ml Tetrahydrofuran gibt man 15,3 g Natriumhydrogencarbonat und tropft unter Eiskühlung bei 10-15°C 18,5 g N-Methyl-N-methoxy-carbaminsäurechlorid hinzu. Nach 6 Stunden Rühren bei 25°C wird filtriert, das Filtrat wird unter vermindertem Druck eingedampft. Man erhält ein Öl, das nach Anreiben mit Diisopropylether kristallisiert. Ausbeute: 38,5 g (79 % d.Th.); Fp. 73-74°C.

Beispiel 2

N-[4-(3-phenyl-2-methyl-n-propan-3-on-yl)-phenyl]-N'-methyl-harnstoff

Eine Lösung von 13,25 g 4-(3-phenyl-2-methyl-n-propan-3-on-yl)-phenyl-isocyanat in 20 ml Toluol wird bei 20 bis 25°C zu einer Lösung von 3 g Methylamin in 100 ml Toluol getropft. Nach 6stündigem Rühren bei 40°C wird das

Toluol unter vermindertem Druck abgezogen. Man erhält 13,3 g (90 % d.Th.) eines schwach gelb gefärbten Öls.

Analog werden beispielsweise die folgenden Verbindungen der Formel I synthetisiert.

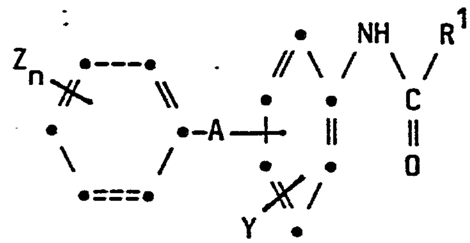

| Nr. | $R^1$ | Stellung von A | A | $Z_n$ | Y | Fp [°C] |
|---|---|---|---|---|---|---|
| 1 | 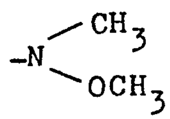 | 3 | $-(CH_2)_2-(CO)-$ | H | H | 91- 92 |
| 2 | " | 3 | $-CO-(CH_2)_2-$ | H | H | 80- 82 |
| 3 | " | 3 | $-CO-(CH_2)_2-$ | 4-Cl | H | 102-103 |
| 4 | " | 4 | $-CO-(CH_2)_2-$ | H | H | |
| 5 | " | 4 | $-CO-(CH_2)_2-$ | $4-CH_3$ | H | 73- 74 |
| 6 | " | 4 | $-CO-(CH_2)_2-$ | 4-Cl | H | 107-109 |
| 7 | " | 4 | $-CO-(CH_2)_2-$ | $2,4,6-(CH_3)_3$ | H | 80- 82 |
| 8 | " | 4 | $-CO-CH(CH_3)-CH_2-$ | H | H | 79- 81 |
| 9 | $NHCH_3$ | 4 | $-CO-CH(CH_3)-CH_2-$ | H | H | Öl |
| 10 | $N(CH_3)_2$ | 4 | $-CO-CH(CH_3)-CH_2-$ | H | H | |
| 11 | 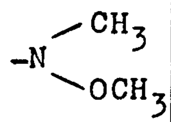 | 4 | $-CO-CH(CH_3)-CH_2-$ | 4-Cl | H | Öl |
| 12 | " | 4 | $-CO-(CH_2)_2-CH(CH_3)-CH_2-$ | $4-CH_3$ | H | 86- 88 |
| 13 | " | 3 | $-CO-(CH_2)_2-CH(CH_3)-CH_2-$ | $4-CH_3$ | H | 76- 78 |
| 14 | " | 4 | $-(CH_2)_2-CO-$ | 4-Cl | H | 124-126 |
| 15 | " | 4 | $-(CH_2)_2-CO-$ | $4-CH_3$ | H | 100-102 |
| 16 | " | 3 | $-CO-(CH_2)_2-$ | $4-OCH_3$ | H | Öl |
| 17 | " | 3 | $-CO-(CH_2)_2-$ | 4-F | H | Öl |
| 18 | " | 3 | $-(CH_2)_2-CO-$ | $3-CF_3$ | H | |

| Nr. | $R^1$ | Stellung von A | A | $Z_n$ | Y | Fp [°C] |
|---|---|---|---|---|---|---|
| 19 | 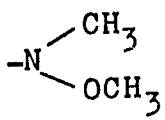 | 3 | $-(CH_2)_2-CO-$ | $3,4-Cl_2$ | H | 112-114 |
| 20 | $C_2H_5$ | 3 | $-(CH_2)_2-CO-(CH_2)_2-$ | H | H | Öl |
| 21 | " | 3 | $-(CH_2)_2-CO-(CH_2)_2-$ | $4-CH_3$ | H | Öl |
| 22 | " | 4 | $-(CH_2)_4-CO-(CH_2)_2-$ | H | H | 102-105 |
| 23 | 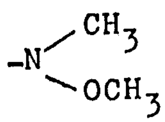 | 4 | $-(CH_2)_4-CO-(CH_2)_2-$ | H | H | Öl |
| 24 | " | 4 | $-(CH_2)_2-CO-(CH_2)_2-$ | $4-CH_3$ | H | Öl |
| 25 | " | 4 | $-(CH_2)_4-CO-(CH_2)_2-$ | H | H | Öl |
| 26 | " | 3 | $-(CH_2)_2-CO-(CH_2)_2-$ | H | H | Öl |
| 27 | " | 3 | $-(CH_2)_2-CO-(CH_2)_2-$ | $4-CH_3$ | H | Öl |
| 28 | " | 4 | $-CH(OH)-(CH_2)_2-$ | $4-CH_3$ | H | Öl |
| 29 | " | 4 | $-CH(OH)-(CH(CH_3)-CH_2-$ | 4-Cl | H | 106-111 |
| 30 | " | 4 | $-(CH_2)_2-CH(OH)-(CH_2)_2-$ | H | H | Öl |
| 31 | " | 3 | $-(CH_2)_4-CH(OH)-$ | H | H | Öl |
| 32 | $C_2H_5$ | 3 | $-CH(OH)-(CH_2)_2-$ | H | H | Öl |
| 33 | | 3 | $-CH(OH)-(CH_2)_2-$ | $4-CH_3$ | H | |
| 34 | " | 3 | $-CH(OH)-(CH_2)_2-$ | 4-F | H | Öl |
| 35 | $OCH_3$ | 3 | $-CO-(CH_2)_2-$ | 4-Cl | H | |
| 36 | $OC_2H_5$ | 3 | $-CO-(CH_2)_2-$ | 4-Cl | H | |

| Nr. | $R^1$ | Stellung von A | A | $Z_n$ | Y | Fp [°C] |
|---|---|---|---|---|---|---|
| 37 | $SCH_3$ | 3 | $-CO-(CH_2)_2-$ | 4-Cl | H | 123-125 |
| 38 | Cyclopropyl | 3 | $-CO-(CH_2)_2-$ | 4-Cl | H | |
| 39 | $C_2H_5$ | 3 | $-CO-(CH_2)_2-$ | 4-Cl | H | 83- 85 |
| 40 | Cyclopropyl-amino | 3 | $-CO-(CH_2)_2-$ | 4-Cl | H | |
| 41 | 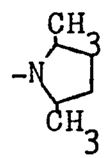 | 3 | $-CO-(CH_2)_2-$ | 4-Cl | H | |
| 42 | $-NHCH_3$ | 3 | $-CO-(CH_2)_2-$ | 4-Cl | H | |
| 43 | $OCH_3$ | 4 | $-CH(OH)-(CH_2)_2-$ | $4-CH_3$ | H | |
| 44 | $SCH_3$ | 4 | $-CH(OH)-(CH_2)_2-$ | $4-CH_3$ | H | |
| 45 | $C_2H_5$ | 3 | $-CO-(CH_2)_2-$ | 4-Cl | 4-Cl | |
| 46 | $C_2H_5$ | 3 | $-CO-(CH_2)_2-$ | 4-Cl | 4-Br | |
| 47 | 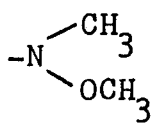 | 4 | $-CO-(CH_2)_3-$ | $4-CH_3$ | 3-Cl | |
| 48 | " | 4 | $-CO-(CH_2)_3-$ | $4-CH_3$ | $3-CH_3$ | |
| 49 | " | 4 | $-CO-(CH_2)_3-$ | $4-CH_3$ | $3-OCH_3$ | |
| 50 | " | 4 | $-CO-(CH_2)_3-$ | $4-CH_3$ | $3-CF_3$ | |

| Nr. | $R^1$ | Stellung von A | A | $Z_n$ | Y | Fp [°C] |
|---|---|---|---|---|---|---|
| 51 | Morpholin-4-yl | 3 | $-CO-(CH_2)_2-$ | 4-Cl | H | |
| 52 | 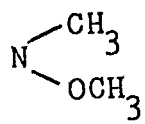 | 4 | $-CO-(CH_2)_2-$ | 3-Cl | H | |
| 53 | " | 4 | $-CO-(CH_2)_2-$ | $4-CF_3$ | H | |
| 54 | $OCH_3$ | 3 | $-CO-(CH_2)_2-$ | H | H | 104-106 |
| 55 | $OCH_3$ | 3 | $-CH(OH)-(CH_2)_2-$ | H | H | 76- 78 |
| 56 | $OCH_3$ | 3 | $-(CH_2)_2-CO-$ | H | H | 115-117 |
| 57 | 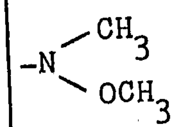 | 4 | $-CH(OH)-(CH_2)_2-$ | H | $4-CF_3$ | |
| 58 | " | 3 | $-CO-(CH_2)_2-$ | H | 4-Br | |
| 59 | " | 3 | $-CO-(CH_2)_2-$ | $2-CH_3$ | H | 84- 86 |
| 60 | " | 3 | $-CO-(CH_2)_2-$ | $3-CH_3$ | H | |
| 61 | " | 4 | $-(CH_2)_2-CO-$ | $3-OC_6H_5$ | H | 81 |
| 62 | " | 4 | $-CO-(CH_2)_2-$ | $4-C_6H_5$ | H | 122-123 |
| 63 | " | 4 | $-CO-(CH_2)_2-$ | $4-O-n-C_6H_{13}$ | H | |
| 64 | " | 3 | $-CH_2-CO-$ | $4-CH_3$ | H | |
| 65 | " | 3 | $-CH_2-CH(OH)-$ | $4-CH_3$ | H | |
| 66 | " | 4 | $-CO-CH_2-$ | $4-CH_3$ | H | |
| 67 | " | 4 | $-CH(OH)-CH_2-$ | $4-CH_3$ | H | |

| Nr. | $R^1$ | Stellung von A | A | $Z_n$ | Y | Fp [°C] |
|---|---|---|---|---|---|---|
| 68 | 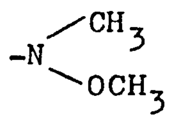 | 4 | $-CO-(CH_2)_3-$ | $4-CH_3$ | H | |
| 69 | " | 4 | $-CH(OH)-(CH_2)_3-$ | $4-CH_3$ | H | |
| 70 | " | 4 | $-CO-CH_2-CH(CH_3)-$ | $4-CH_3$ | H | |
| 71 | " | 3 | $-CH(CH_3)-CH_2-CO-$ | $4-CH_3$ | H | |
| 72 | " | 4 | $-C(CH_3)_2-CH_2-CO-$ | H | H | |
| 73 | " | 3 | $-C(CH_3)_2-CH_2-CO-$ | H | H | |
| 74 | " | 4 | $-CO-(CH_2)_5-$ | H | H | |
| 75 | " | 4 | $-CO-(CH_2)_7-$ | H | H | |
| 76 | " | 4 | $-CO-(CH_2)_8-$ | H | H | |
| 77 | $N(CH_3)_2$ | 3 | $-(CH_2)_2-CO-$ | H | H | 88- 90 |
| 78 | 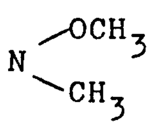 | 4 | $-(CH_2)_2-CO-$ | $3-CF_3$ | H | 108-109 |
| 79 | " | 4 | $-CH_2-CH(CH_3)-(CH_2)_2-CO-$ | H | H | 65- 67 |
| 80 | " | 4 | $-(CH_2)_2-CO-$ | 3-Cl | H | 98-100 |
| 81 | 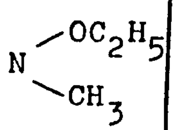 | 4 | $-CO-(CH_2)_2-$ | $4-CH_3$ | H | 71- 72 |
| 82 | " | 4 | $-CH(OH)(CH_2)_2-$ | $4-CH_3$ | H | 63- 64 |

| Nr. | $R^1$ | Stellung von A | A | $Z_n$ | Y | Fp [°C] |
|---|---|---|---|---|---|---|
| 90 | 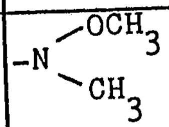 | 4 | $-(CH_2)_2-O-$ | $4-t-OC_4H_9$ | H | 86- 87 |
| 91 | " | 3 | $-CO-(CH_2)_2-$ | $4-CH_3$ | H | 90- 92 |
| 92 | $SCH_3$ | 3 | $-CO-(CH_2)_2-$ | $4-CH_3$ | H | 145-147 |
| 93 | $C_2H_5$ | 3 | $-CO-(CH_2)_2-$ | $4-CH_3$ | H | 86- 88 |
| 94 | 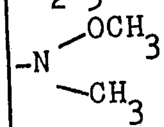 | 3 | $-CH(OH)-(CH_2)_2-CH(CH_3)-CH_2-$ | H | H | Öl |
| 95 | $C_2H_5$ | 3 | $-CH_2-CO-$ | $4-CF_3$ | H | 124-126 |
| 96 | 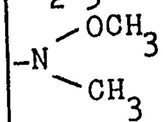 | 3 | $-CO-(CH_2)_2-$ | $4-CH_3$ | H | 112-113 |
| 97 | " | 3 | $-CO-(CH_2)_2-$ | $4-CH_3$ | H | 90- 92 |
| 98 | " | 4 | $-(CH_2)_2-CH(OH)-$ | $4-CH_3$ | H | 107-108 |
| 99 | " | 4 | $-CH(OH)-CH(CH_3)-CH_2-$ | H | H | Öl |
| 100 | " | 3 | $-CO-(CH_2)_2-$ | $2,4,6-(CH_3)_3$ | H | Öl |
| 101 | $C_2H_5$ | 3 | $-CO-(CH_2)_2-$ | $2,4,6-(CH_3)_3$ | H | 80- 81 |
| 102 | $OCH_3$ | 3 | $-CO-(CH_2)_2-$ | $2,4,6-(CH_3)_3$ | H | 57- 58 |
| 103 | $SCH_3$ | 3 | $-CO-(CH_2)_2-$ | $2,4,6-(CH_3)_3$ | H | 110-112 |
| 104 | $N(CH_3)_2$ | 3 | $-CO-(CH_2)_2-$ | $2,4,6-(CH_3)_3$ | H | 151-153 |
| 105 | 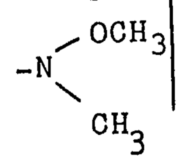 | 4 | $-CO-(CH_2)_2-$ | 4-F | H | 94- 96 |

| Nr. | $R^1$ | Stellung von A | A | Zn 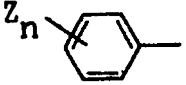 | Y | Fp [°C] |
|---|---|---|---|---|---|---|
| 83 | $C_2H_5$ | 3 | $-CO-(CH_2)_2-$ | β-Naphthyl | H | |
| 84 | $C_2H_5$ | 3 | $-CO-(CH_2)_2-$ | α-Naphthyl | H | |
| 85 | $-N(OCH_3)(CH_3)$ | 4 | $-CO-(CH_2)_2-$ | β-Naphthyl | H | 91-93 |
| 86 | " | 4 | $-CO-(CH_2)_2-$ | α-Naphthyl | H | 113-115 |
| 87 | " | 3 | $-CO-(CH_2)_2-$ | β-Naphthyl | H | |
| 88 | " | 3 | $-CO-(CH_2)_2-$ | α-Naphthyl | H | |
| 89 | " | 4 | $-CO-(CH_2)_2-$ | 4-Chlor-α-naphthyl | H | 61- 63 |

Die Verbindungen der Formel I oder deren Säureadditionssalze können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende

Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat,

Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile der Verbindung Nr. 1 mit 10 Gewichtsteilen N-Methyl-$\alpha$-pyrrolidon und erhält eine Lösung, die zur Anwendung in Form kleinster Tropfen geeignet ist.

II. 20 Gewichtsteile der Verbindung Nr. 3 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 5 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteile des Anlagerungsproduktes von 40 Mol Ethylenoxid an 1 Mol Ricinusöl besteht. Durch Eingießen und feines Verteilen der Lösung in 100 000 Gewichtsteilen Wasser

erhält man eine wäßrige Dispersion, die 0,02 Gewichtsprozent des Wirkstoffs enthält.

IV. 20 Gewichtsteile der Verbindung Nr. 3 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile der Verbindung Nr. 1 werden mit 3 Gewichtsteilen des Natriumsalzes der Diisobutylnaphthalin- -sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile der Verbindung Nr. 11 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile der Verbindung Nr. 13 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 17 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der Mittel kann im Vorauflaufverfahren oder im Nachauflaufverfahren erfolgen. Vorzugsweise werden die neuen Wirkstoffe nach dem Auflaufen der unerwünschten Pflanzen, sowohl auf Kulturflächen als auch auf unbebautem Land, ausgebracht. Sind die Wirkstoffe für die Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Bekämpfungsziel und Wachstumsstadium 0,05 bis 10 kg/ha und mehr, vorzugsweise 0,1 bis 5 kg/ha.

Die herbizide Wirkung von Verbindungen der Formel I wird durch Gewächshausversuche gezeigt:

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät. Unmittelbar danach erfolgt bei Vorauflaufbehandlung das Aufbringen der Wirkstoffe auf die Erdoberfläche. Sie werden hierbei in Wasser als Verteilungsmittel suspendiert oder emulgiert und mittels fein ver-

teilender Düsen gespritzt. Bei dieser Applikationsmethode beträgt die Aufwandmenge 3,0 Wirkstoff/ha. Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Die Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform bis zu einer Wuchshöhe von 3 bis 15 cm an und behandelt sie danach. Die für die Nachauflaufanwendung benutzten Sojapflanzen zieht man in einem mit Torfmull (peat) angereichertem Substrat an. Zur Nachauflaufbehandlung werden entweder direkt gesäte und in den gleichen Gefäßen aufgewachsenen Pflanzen ausgewählt, oder sie werden erst als Keimpflanzen getrennt angezogen und einige Tage vor der Behandlung in die Versuchsgefäße verpflanzt. Die Aufwandmengen für die Nachauflaufbehandlung variieren je nach Wirkstoff. Sie betragen 0,25 oder 0,5 kg Wirkstoff/ha. Die Abdeckung unterbleibt bei der Nachauflaufbehandlung.

Die Versuchsgefäße werden im Gewächshaus aufgestellt, wobei für wärmeliebende Arten wärmere Bereiche (20 bis 35°C) und für solche gemäßigter Klimate 10 bis 25°C bevorzugt wurden. Die Versuchsperiode erstreckt sich über 2 bis 4 Wochen. Während dieser Zeit werden die Pflanzen gepflegt, und ihre Reaktion auf die einzelnen Behandlungen ausgewertet. Bewertet wird nach einer Skala von 0 bis 100. Dabei bedeutet 0 keine Schädigung oder normaler Ablauf und 100 kein Aufgang der Pflanzen bzw. völlige Zerstörung zumindest der oberirdischen Teile.

Die in den Versuchen verwendeten Pflanzen stammen von folgenden Arten:

Amaranthus retroflexus (zurückgekrümmter Fuchsschwanz), Avena sativa (Hafer), Abutilon theophrasti (Chinesischer Hanf), Arachys hypogaea (Erdnüsse), Chenopodium album (Weißer Gänsefuß), Cassia tora, Gossypium hirsutum (Baumwolle), Glycine max. (Soja), Euphorbia geniculata (Südamerikanische Wolfsmilchart), Lamium pupureum (Taubnessel), Sida spinosa, Sinapis alba (weißer Senf), Solanum nigrum (Schwarzer Nachschatten), Triticum aestivum (Weizen), Zea mays (Mais).

Bei Nachauflaufanwendung zeigen beispielsweise die Verbindungen Nr. 1, 3, 5, 7, 11, 13, 17 und 79 eine gute herbizide Wirkung, insbesondere gegen unerwünschte breitblättrige Pflanzen; sie sind gleichzeitig selektiv gegenüber Kulturpflanzen.

Eine herbizide Vorauflaufaktivität haben beispielsweise die Verbindung Nr. 1 an weißem Senf und die Verbindung Nr. 5 an Hafer.

In Anbetracht der guten Verträglichkeit der Wirkstoffe und der Vielseitigkeit der Applikationsmethoden können die erfindungsgemäßen Verbindungen oder diese enthaltende Mittel außer bei den in den Gewächshausversuchen getesteten Nutzpflanzen noch in einer weiteren großen Zahl von Kulturpflanzen zur Beseitigung unerwünschten Pflanzenwuchses eingesetzt werden.

In Betracht kommen beispielsweise die folgenden Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum Gossypium herbaceum Gossypium vitifolium) | Baumwolle |
| Helianthus annuus | Sonnenblume |

| Botanischer Name | Deutscher Name |
|---|---|
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersiocon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- u. Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |

| Botanischer Name | Deutscher Name |
|---|---|
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium carymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die neuen erfindungsgemäßen Verbindungen mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem kann es von Nutzen sein, die Verbindungen der Formel I bzw. sie enthaltende herbizide Mittel allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

Patentansprüche

1. Anilinderivate der Formel

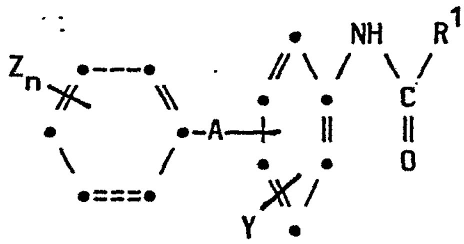

(I),

in der

$R^1$ Alkoxy, Alkylthio oder gegebenenfalls durch Halogen oder Alkoxy mit 1 bis 4 Kohlenstoffatomen substituiertes Alkyl mit jeweils bis zu 4 Kohlenstoffatomen oder Cycloalkyl mit 3 bis 6 Kohlenstoffatomen oder den Rest $-N<^{R^2}_{R^3}$, wobei $R^2$ und $R^3$ unabhängig voneinander Wasserstoff, Alkyl oder Alkoxy mit jeweils bis zu 4 Kohlenstoffatomen oder Cycloalkyl mit 3 bis 6 Kohlenstoffatomen bedeuten oder $R^2$ und $R^3$ zusammen eine gegebenenfalls durch Methyl substituierte und gegebenenfalls Sauerstoff als Kettenglied enthaltende Alkylenkette mit bis zu 6 Kohlenstoffatomen bedeuten,

Y Wasserstoff, Halogen, Methyl, Methoxy oder Trifluormethyl,

A eine die Carbonyl- oder die Carbinolgruppe der Formel $>CH-OH$ enthaltende Alkylenkette mit 2 bis 9 Kohlenstoffatomen, die durch Methyl substituiert sein kann,

Z Wasserstoff, Halogen, Alkyl, Alkoxy, Halogenalkyl oder Halogenalkoxy mit jeweils bis zu 6 Kohlenstoffatomen, Phenyl oder Phenoxy und

n die Zahlen 1, 2 oder 3 bedeuten und in der anstelle des Restes 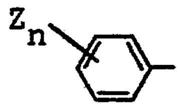 ein gegebenenfalls durch Halogen, Alkyl oder Alkoxy mit jeweils bis zu 4 Kohlenstoffatomen substituierter Naphthylrest stehen kann.

2. Anilinderivate der Formel I gemäß Anspruch 1, <u>dadurch gekennzeichnet</u>, daß $R^1$ den Rest 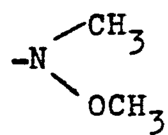, Y Wasserstoff oder Halogen, A den Rest $-CO-(CH_2)_2-$ in para-Stellung zu der Gruppe $-NH-CO-R^1$, Z Halogen oder Alkyl mit bis zu 4 Kohlenstoffatomen und n 1 bedeuten.

3. Anilinderivate der Formel I gemäß Anspruch 1, <u>dadurch gekennzeichnet</u>, daß $R^1$ den Rest 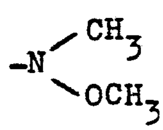, Y Wasserstoff oder Chlor, A den Rest $-CO-(CH_2)_2-$ in para-Stellung zu der Gruppe $-NH-CO-R^1$, Z Chlor oder Methyl und n 1 bedeuten.

4. N-[4-(3-(2',4',6'-trimethylphenyl)-n-propan-3-on-yl)-phenyl]-N'-methoxy-N'-methyl-harnstoff.

5. Verfahren zur Herstellung der Anilinderivate der Formel I gemäß Anspruch 1, <u>dadurch gekennzeichnet</u>, daß man ein Amin der Formel

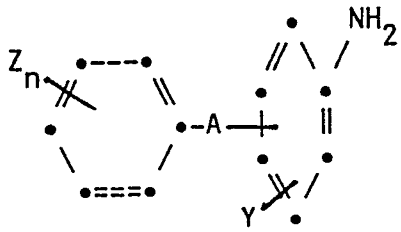
(II),

in der A, Y, Z und n die im Anspruch 1 genannten Bedeutungen haben, mit einer Verbindung der Formel

$$R^1 - CO - X \qquad (III),$$

in der $R^1$ die im Anspruch 1 angegebenen Bedeutungen hat und X eine Abgangsgruppe oder den Rest $R^1$-CO-O- bedeutet, in Gegenwart eines inerten organischen Lösungsmittels und eines Säureakzeptors bei einer Temperatur im Bereich zwischen 20 und 80°C umsetzt.

6. Herbizid, enthaltend inerte Zusatzstoffe und ein Anilinderivat der Formel

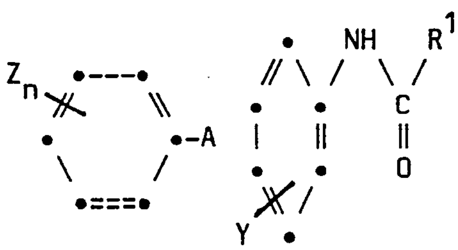
(I),

in der

$R^1$ Alkoxy, Alkylthio oder gegebenenfalls durch Halogen oder Alkoxy mit 1 bis 4 Kohlenstoffatomen substituiertes Alkyl mit jeweils bis zu 4 Kohlenstoffatomen oder Cycloalkyl mit 3 bis 6 Kohlenstoffatomen oder den Rest $-N\genfrac{}{}{0pt}{}{R^2}{R^3}$, wobei $R^2$

und $R^3$ unabhängig voneinander Wasserstoff, Alkyl oder Alkoxy mit jeweils bis zu 4 Kohlenstoffatomen oder Cycloalkyl mit 3 bis 6 Kohlenstoffatomen bedeuten oder $R^2$ und $R^3$ zusammen eine gegebenenfalls durch Methyl substituierte und gegebenenfalls Sauerstoff als Kettenglied enthaltende Alkylenkette mit bis zu 6 Kohlenstoffatomen bedeuten,

Y Wasserstoff, Halogen, Methyl, Methoxy oder Trifluormethyl,

A eine die Carbonyl- oder die Carbinolgruppe der Formel >CH-OH enthaltende Alkylenkette mit 2 bis 9 Kohlenstoffatomen, die durch Methyl substituiert sein kann,

Z Wasserstoff, Halogen, Alkyl, Alkoxy, Halogenalkyl oder Halogenalkoxy mit jeweils bis zu 6 Kohlenstoffatomen, Phenyl oder Phenoxy und

n die Zahlen 1, 2 oder 3 bedeuten und in der anstelle des Restes 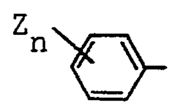 ein gegebenenfalls durch Halogen, Alkyl oder Alkoxy mit jeweils bis zu 4 Kohlenstoffatomen substituierter Naphthylrest stehen kann.

7. Herbizid, enthaltend inerte Zusatzstoffe und ein Anilinderivat der Formel I gemäß Anspruch 1, <u>dadurch gekennzeichnet</u>, daß $R^1$ den Rest 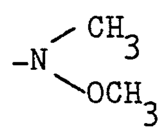, Y Wasserstoff oder Halogen, A den Rest $-CO-(CH_2)_2-$ in para-Stellung zu der Gruppe $-NH-CO-R^1$, Z Halogen oder Alkyl mit bis zu 4 Kohlenstoffatomen und n 1 bedeuten.

8. Verfahren zur Bekämpfung unerwünschten Pflanzenwuchses, dadurch gekennzeichnet, daß man eine herbizid wirksame Menge eines Anilinderivates der Formel I gemäß Anspruch 1 auf die Pflanzen und/oder ihren Standort einwirken läßt.

9. Amine der Formel

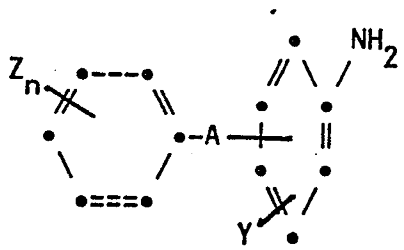

(II),

in der

A eine durch die Carbonyl- oder durch die Carbinolgruppe der Formel >CH-OH unterbrochene Alkylenkette mit 2 bis 9 Kohlenstoffatomen, die gegebenenfalls durch Methyl substituiert sein kann,

Y Wasserstoff, Halogen, Methyl, Methoxy oder Trifluormethyl,

Z Wasserstoff, Halogen, Alkyl, Alkoxy, Halogenalkyl oder Halogenalkoxy mit jeweils bis zu 6 Kohlenstoffatomen, Phenyl oder Phenoxy und

n die Zahlen 1, 2 oder 3 bedeuten und in der anstelle des Restes 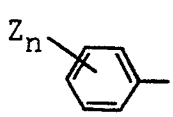 ein gegebenenfalls durch Halogen, Alkyl oder Alkoxy mit jeweils bis zu 4 Kohlenstoffatomen substituierter Naphthylrest stehen kann.

10. Verwendung von Aminen der Formel

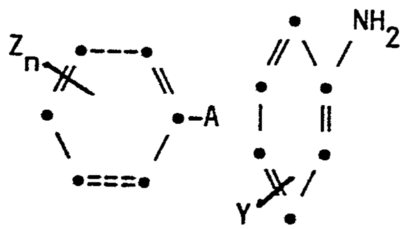

(II),

in der

A eine durch die Carbonyl- oder durch die Carbinolgruppe der Formel >CH-OH unterbrochene Alkylenkette mit 2 bis 9 Kohlenstoffatomen, die gegebenenfalls durch Methyl substituiert sein kann,

Y Wasserstoff, Halogen, Methyl, Methoxy oder Trifluormethyl,

Z Wasserstoff, Halogen, Alkyl, Alkoxy, Halogenalkyl oder Halogenalkoxy mit jeweils bis zu 6 Kohlenstoffatomen, Phenyl oder Phenoxy und

n die Zahlen 1, 2 oder 3 bedeuten und in der anstelle des Restes 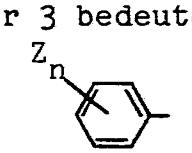 ein gegebenenfalls durch Halogen, Alkyl oder Alkoxy mit jeweils bis zu 4 Kohlenstoffatomen substituierter Naphthylrest stehen kann,

zur Herstellung von Anilinderivaten der Formel I gemäß Anspruch 1.